# EUROPEAN PATENT APPLICATION

(11) **EP 4 286 850 A1**
(43) Date of publication of application: **06.12.2023**
(21) Application number: 22745826.2
(22) Date of filing: 25.01.2022
(51) Int. Cl.: G01N 33/543

(54) **IMMUNOLOGICAL ASSAY METHOD**

(30) Priority: 26.01.2021 JP 2021010184
(71) Applicant: Sekisui Medical Co., Ltd., Tokyo 103-0027 (JP)
(72) Inventor: FUJIMURA Kengo, Tokyo 103-0027 (JP); NOMOTO Daigo, Tokyo 103-0027 (JP); SHIMIZU Tomo, Tokyo 103-0027 (JP); KORA Yuki, Tokyo 103-0027 (JP)
(74) Representative: Hoffmann Eitle
(86) International application number: PCT/JP2022/002518
(87) International publication number: WO 2022/163605

(57) **Abstract**

An object of the invention is to suppress non-specific reaction in an immunoreaction measurement method. An immunoassay method characterized by conducting immunoreaction in the presence of an anti-C3 antibody in a method for measuring an analyte in a sample immunologically is provided. Moreover, a method for suppressing non-specific reaction in a method for immunologically measuring an analyte in a sample which is characterized by conducting immunoreaction in the presence of an anti-C3 antibody is provided.

## Description

### TECHNICAL FIELD

The present invention relates to an immunoassay. In particular, the invention relates to an immunoassay in which immunoreaction is conducted in the presence of an anti-C3 antibody.

### BACKGROUND ART

Measurement methods in the field of diagnostic agents include an immunoassay, which measures an analyte which exists in a biological sample using immunoreaction. The immunoassay uses antigen-antibody reaction and thus the specificity of this method is very high.

Various substances are contained in a biological sample, and substances that cause non-specific reaction, which are so-called non-specific factors (factors of interference / factors of non-specific reaction / non-specific binding molecules), are often contained. Non-specific reaction is a phenomenon in which binding that is not based on specific reaction is promoted or in which specific immunoreaction is inhibited, and non-specific reaction causes measurement errors. As non-specific factors, heterophile antibodies and rheumatoid factor (RF) has been known. Heterophile antibody is a general term for human antibodies which show reactivity to an animal-derived antibody which is a main component of an immunoassay, and HAMA (human anti-mouse immunoglobulin antibody) is known as a typical heterophile antibody. Rheumatoid factor emerges in patients with rheumatoid arthritis and is similar to HAMA in terms of the property of showing reactivity to an animal-derived antibody, and the identity of rheumatoid factor and HAMA is known to be actually human immunoglobulin G or immunoglobulin M (Non-Patent Document 1).

As a technique for suppressing non-specific reaction in an immunoassay, for example, Patent Document 1 is known. Patent Document 1 discloses a method for suppressing non-specific reaction caused by rheumatoid factor (RF) by pre-treating the sample in advance with a sufficient amount of an animal-derived antibody capable of binding to the reaction site of RF. As the animal-derived antibody, an anti-human IgG antibody, an anti-human IgA antibody and/or an anti-human IgM antibody are disclosed.

### CITATION LIST

### PATENT LITERATURE

Patent Document 1: JP-A-07-012818

### NON PATENT LITERATURE

Non-Patent Document 1: Clinical Chemistry, Vol. 23, Supplementary Book, 175a-1 to 175a-10 (1994)

### SUMMARY OF INVENTION

### TECHNICAL PROBLEM

Patent Document 1 describes a method for suppressing non-specific reaction caused by a natural antibody or RF with an anti-human IgM antibody, an anti-human IgG antibody or an anti-human IgA antibody. This is a method which is currently used practically with various reagents, but the tests conducted by the present inventors have revealed that there is non-specific reaction which cannot be suppressed even with these antibodies. An object of the invention is to suppress non-specific reaction in an immunoassay method.

### SOLUTION TO PROBLEM

To solve the problem, the inventors of the present application have examined the effects of various substances of suppressing non-specific reaction and found as a result that non-specific reaction can be suppressed by conducting immunoreaction in the presence of an anti-C3 antibody, and the invention of the present application has been thus completed. That is, the invention of the present application has the following structures.

Here, anti-C3 antibodies are known to be used for detecting a substance-to-be-measured (C3 itself is the analyte) in an immunoassay, but the effect of suppressing non-specific reaction in immunoreaction is not known at all.
<1> An immunoassay method comprising conducting immunoreaction in the presence of an anti-C3 antibody in a method for measuring an analyte in a sample immunologically.
<2> The immunoassay method according to <1>, wherein the immunoassay method is a method based on a homogeneous method or a heterogeneous method.
<3> The immunoassay method according to <1> or <2>, wherein the method based on a homogeneous method is a latex immunoagglutination measurement method.
<4> The immunoassay method according to <3>, comprising
   a step of bringing the analyte in the sample and the anti-C3 antibody into contact with each other in a solution,
   a step of adding latex particles carrying a specific binding partner for the analyte to the solution and
   a step of optically detecting the degree of agglutination of the latex particles in the solution.
<5> The immunoassay method according to any of <1> to <4>, wherein the anti-C3 antibody is any one or more selected from the group consisting of an anti-iC3b antibody, an anti-C3dg antibody, an anti-C3d antibody and an anti-C3b antibody.
<6> A method for suppressing non-specific reaction in a method for measuring an analyte in a sample immunologically, comprising conducting immunoreaction in the presence of an anti-C3 antibody.
<7> The method for suppressing non-specific reaction according to <6>, wherein the immunoassay method is a method based on a homogeneous method or a heterogeneous method.
<8> The method for suppressing non-specific reaction according to <6> or <7>, wherein the method based on a homogeneous method is a latex immunoagglutination measurement method.
<9> The method for suppressing non-specific reaction according to <8>, comprising
   a step of bringing the analyte in the sample and the anti-C3 antibody into contact with each other in a solution,
   a step of adding latex particles carrying a specific binding partner for the analyte to the solution and
   a step of optically detecting the degree of agglutination of the latex particles in the solution.
<10> The immunoassay method according to any of <6> to <9>, wherein the anti-C3 antibody is any one or more selected from the group consisting of an anti-iC3b antibody, an anti-C3dg antibody, an anti-C3d antibody and an anti-C3b antibody.
<11> An immunoassay reagent containing an anti-C3 antibody.
<12> The immunoassay reagent according to <11>, wherein a method of the immunoassay is a method based on a homogeneous method or a heterogeneous method.
<13> The immunoassay reagent according to <12>, wherein the method based on a homogeneous method is a latex immunoagglutination assay.
<14> The immunoassay reagent according to any of <11> to <13>, wherein the anti-C3 antibody is any one or more selected from the group consisting of an anti-iC3b antibody, an anti-C3dg antibody, an anti-C3d antibody and an anti-C3b antibody.
<15> An immunoassay reagent kit containing an anti-C3 antibody.
<16> The immunoassay reagent kit according to <15>, wherein a method of the immunoassay is a method based on a homogeneous method or a heterogeneous method.
<17> The immunoassay reagent kit according to <16>, wherein the method based on a homogeneous method is a latex immunoagglutination assay, and the immunoassay reagent kit comprises:
   (1) a first reagent containing the anti-C3 antibody; and
   (2) a second reagent containing latex particles carrying a specific binding partner for an analyte.
<18> The immunoassay reagent according to any of <15> to <17>, wherein the anti-C3 antibody is any one or more selected from the group consisting of an anti-iC3b antibody, an anti-C3dg antibody, an anti-C3d antibody and an anti-C3b antibody.
<19> An agent for suppressing non-specific reaction in an immunoassay method containing an anti-C3 antibody as an active ingredient.
<20> The agent for suppressing non-specific reaction in an immunoassay method according to <19>, wherein the anti-C3 antibody is any one or more selected from the group consisting of an anti-iC3b antibody, an anti-C3dg antibody, an anti-C3d antibody and an anti-C3b antibody.

### ADVANTAGEOUS EFFECTS OF INVENTION

According to the invention, by conducting immunoreaction in the presence of an anti-C3 antibody in an immunoassay method, non-specific reaction due to a non-specific factor contained in a sample can be suppressed, and accurate measurement has become possible. In particular, it has great significance that suppression of non-specific reaction which still could not be suppressed even with a commercial agent for suppressing non-specific reaction has become possible by the invention.

### BRIEF DESCRIPTION OF DRAWINGS

[Fig. 1] Fig. 1 shows a correlation of the measured values of Comparative Example 2 (measured by an LTIA method without the addition of agents for suppressing non-specific reaction) to the measured values of Comparative Example 1 (a CLEIA).
[Fig. 2] Fig. 2 shows a correlation of the measured values of Comparative Example 3 (an LTIA with the addition of HBR-1 as an agent for suppressing non-specific reaction) to the measured values of Comparative Example 1 (a CLEIA).
[Fig. 3] Fig. 3 shows a correlation of the measured values of Example 1 (an LTIA with the addition of the anti-C3d polyclonal antibody of the invention of the present application as an agent for suppressing non-specific reaction) to the measured values of Comparative Example 1 (a CLEIA).
[Fig. 4] Fig. 4 is a schematic figure showing the structure, activation and decomposition of complement protein C3 (cited from Introduction to Complement Science: From Basics to Clinical/Measurement Method, Hajime Kitamura, Interdisciplinary Planning Co., Ltd. (2010.11), Fig. 10 on p27).

### DESCRIPTION OF EMBODIMENTS

### (Immunoassay Method)

The immunoassay method of the invention is a method characterized by conducting immunoreaction in the presence of an anti-C3 antibody in a method for measuring an analyte in a sample immunologically. In other words, in the method, the analyte in the sample is immunologically measured using a specific binding partner other than the anti-C3 antibody in the presence of the anti-C3 antibody as an agent for suppressing non-specific reaction.

Immunoassay methods are further roughly classified into homogeneous methods and heterogeneous methods.

A homogeneous method is a measurement method which specifically detects the reaction that is advanced by the analyte in a mixed solution (reaction solution) of a sample and a reagent liquid without B/F (bound/free) separation, and a heterogeneous method is a measurement method which advances and detects the main reaction after washing/removing excessive components that have not involved in binding reaction by B/F separation operation.

While the heterogeneous methods have a problem because the methods include many steps due to the washing step and require time for the measurement, the heterogeneous methods have an advantage because the methods are relatively less affected by the non-specific reaction substances. On the other hand, while the homogeneous methods have a problem because the methods do not include a washing step and are thus easily affected by non-specific reaction, the homogeneous methods are widely needed in the field of clinical diagnosis because the methods are simple with fewer steps and because the time required for the measurement is short.

The homogeneous methods include a measurement method using an immunoagglutination assay (turbidimetric immunoassay, TIA) and immunochromatography (lateral flow type and flow-through type). A TIA is a method for qualitatively or quantitatively detecting an analyte (a substance-to-be-measured) in a sample based on the degree of agglutination of an immune complex formed through the action of a specific binding partner such as an antibody of crosslinking the analyte, and in particular, a latex immunoagglutination assay (sometimes called an LTIA below), which uses latex particles as an insoluble carrier for amplifying the agglutination signal, is a great general purpose measurement method which is applied to various testing items because the assay is suitable for optical detection and is easy to automize.

The heterogeneous methods include an ELISA using a well plate, chemiluminescent measurement and the like.

The invention can be used in any of the immunoassay methods, but a homogeneous method, which is relatively easily affected by non-specific reaction, is preferable because the benefits are expected to be enjoyed clearly.

### (Anti-C3 Antibody)

The anti-C3 antibody used for suppressing non-specific reaction in the invention is an antibody against C3 and may be any antibody as long as it recognizes a part corresponding to C3d of C3.

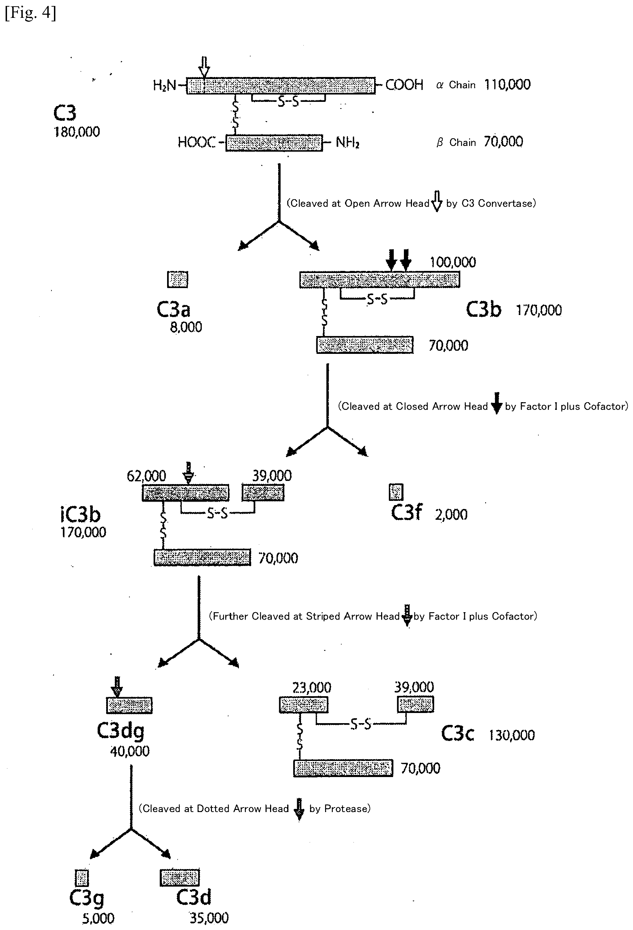

C3 is one of complement proteins in the classical pathway and is also called the complement third component or β1C globulin due to the electrical mobility. C3 is a protein having a molecular weight of 180 kDa, has a structure in which an α chain having a molecular weight of 110 kDa and a β chain having a molecular weight of 70 kDa are crosslinked with a -S-S- bond and is a core protein of the complement system. As shown in Fig. 4, C3 is decomposed into C3dg through C3b and iC3b and further into C3d. The anti-C3 antibody used for the invention may be any antibody as long as it recognizes a part corresponding to C3d, which is generated through decomposition of C3, and in this sense, any of an anti-iC3b antibody, an anti-C3b antibody, an anti-C3dg antibody and an anti-C3d antibody can be used. Of these, an anti-C3d antibody is particularly preferable.

The anti-C3 antibody used for the invention may be a polyclonal antibody or a monoclonal antibody. A monoclonal antibody is more preferable.

As the anti-C3 antibody used for the invention, functional fragments of an antibody having antigen-antibody reactivity can also be used in addition to the whole antibody molecule.

The anti-C3 antibody used for the invention may be an antibody obtained through an immunization step of a general animal (a mouse, a goat, a sheep or the like) or an antibody in which the amino acid sequence has been changed to a sequence of an animal species that is different from the animal immunized with the immunogen (the substance-to-be-measured / analyte) using genetic recombination technique or the like (a chimeric antibody, a humanized antibody, a completely humanized antibody or the like). The functional fragments of the antibody include F(ab')₂ and Fab', which are fragments having antigen-antibody reactivity, as well as a single-chain antibody (scFv), a VHH antibody (variable domain of heavy chain of heavy chain antibody), a IgNAR (new antigen receptor) antibody and the like. The functional fragments of the antibody can be produced by treating an antibody obtained as described above with a protease (for example, pepsin, papain or the like). In the invention, the functional fragments of the anti-C3 antibody may be anything as long as the fragments contain a part corresponding to C3d as described above. In the invention, unless otherwise specified, the anti-C3 antibody also includes functional fragments.

As the antibody against C3 used for the invention, any one of the group consisting of an anti-C3b antibody, an anti-iC3b antibody, an anti-C3dg antibody and an anti-C3d antibody can be used, and a combination of any two or more kinds selected from the group can also be used.

In the present specification, that an antibody "reacts with" an antigen and that an antibody "recognizes" an antigen are used synonymously but should not be limited to the examples and should be construed in the broadest sense. Whether an antibody "reacts with" an antigen (compound) or not can be checked by an antigen-immobilized ELISA, a competitive ELISA, a sandwich ELISA or the like and can also be checked by a method using the principle of surface plasmon resonance (SPR method) or the like. The SPR method can be conducted using a device, a sensor and reagents which are commercially available under the name of Biacore (registered trademark).

The antibody against C3 used for the invention can be produced by dissolving C3 such as a commercial human-derived C3d protein as an antigen (immunogen) in a solvent such as phosphate-buffered physiological saline and administering the solution to an animal for immunization. When necessary, an appropriate adjuvant may be added to the solution, and then an animal may be immunized using the emulsion. As the adjuvant, generally used adjuvants such as water-in-oil emulsion, water-in-oil-in-water emulsion, oil-in-water emulsion, liposome and aluminum hydroxide gel as well as proteins and peptide substances derived from biological components and the like may be used. For example, incomplete Freund's adjuvant, complete Freund's adjuvant or the like can be suitably used. The administration route, the dosage and the timing of administration of the adjuvant are not particularly limited but are desirably selected appropriately so that desired immune response can be enhanced in the animal immunized with the antigen.

The kind of animal used for immunization is not particularly limited, either, but is preferably a mammal. For example, mice, rats, cows, rabbits, goats, sheep, alpacas and the like can be used, and mice or rats can be used more preferably. An animal may be immunized according to a general method and can be immunized, for example, by injecting a solution of the antigen, preferably a mixture with an adjuvant, subcutaneously, intracutaneously, intravenously or intraperitoneally to the animal. Because the immune response generally differs with the kind and the line of the animal to be immunized, the immunization schedule is desirably set appropriately depending on the animal used. The administration of the antigen is preferably repeated several times after the first immunization.

To obtain monoclonal antibodies, the following operations are subsequently performed, although the method is not limited to this. The method for producing a monoclonal antibody itself is known in the field and is generally used, and thus one skilled in the art can easily produce the antibody used in the immunological analysis method of the invention using the antigen (for example, see Antibodies, A Laboratory Manual (Cold Spring Harbor Laboratory Press, (1988), section 6 and the like).

After the final immunization, spleen cells or lymph node cells, which are antibody-producing cells, are taken out of the immunized animal, and hybridomas can be produced by cell fusion with a myeloma-derived cell line having high growth potential. Cells with high antibody-producing potential (quality/amount) are preferably used for the cell fusion, and the myeloma-derived cell line preferably has compatibility with the animal from which the antibody-producing cells to be fused are derived. The cells can be fused according to a method known in the field, but for example, polyethylene glycol method, a method using Sendai virus, a method using current and the like can be employed. The obtained hybridomas can be proliferated according to conditions generally used in the field, and a desired hybridoma can be selected while the properties of the produced antibody are examined. The hybridoma can be cloned by a known method such as limiting dilution analysis or soft agar analysis, for example.

The hybridoma can also be selected efficiently at the selection stage considering the conditions under which the produced antibody is used for actual measurement. For example, a hybridoma which produces a desired antibody can be selected more efficiently by reacting an antibody obtained by immunization of an animal with C3 immobilized on a solid phase in the presence of a compound whose cross-reactivity is to be examined and comparing the reactivity with the reactivity in the absence of the compound whose cross-reactivity is to be examined. Moreover, a hybridoma which produces a desired antibody can also be selected more efficiently by reacting an antibody obtained by immunization of an animal with C3 immobilized on a solid phase in the presence of a component derived from a biological sample and comparing the reactivity with the reactivity in the absence of the component derived from the biological sample.

After the cloning step, whether the selected hybridoma produces a monoclonal antibody having desired properties or not can be examined through an assay of the binding potential of the produced antibody and C3 using a method such as an ELISA, an RIA and a fluorescent antibody method.

By culturing the hybridoma which is selected as described above in a large scale, the monoclonal antibody having desired characteristics can be produced. The large-scale culture method is not particularly limited, but examples thereof include a method for culturing the hybridoma in an appropriate medium and thus producing the monoclonal antibody in the medium, a method for intraperitoneally injecting the hybridoma to a mammal, proliferating the hybridoma and producing the antibody in the ascites and the like. The monoclonal antibody can be purified by an appropriate combination of the methods for purifying an antibody from antiserum described above such as DEAE anion-exchange chromatography, affinity chromatography, ammonium sulfate fractionation, PEG fractionation and ethanol fractionation, for example.

The method for making the anti-C3 antibody to be present in the immunoreaction system may be a method using the anti-C3 antibody as one of the components of the immunoassay reagent or a method for adding to a diluent or a pretreatment liquid for the sample or the like.

When the immunoassay reagent is a liquid reagent, for example, an immunoreaction system (or being in a reaction system) refers to a liquid phase (or being in a liquid phase) in which the sample and the liquid immunoassay reagent are mixed and in which the immunoreaction is conducted.

For example, in the case of an LTIA, the sample may be mixed with an LTIA measurement reagent containing the anti-C3 antibody, or the sample is mixed in advance with a pretreatment liquid containing the anti-C3 antibody and then mixed with the immunoassay reagent.

In the case of an ELISA, the sample may be mixed in advance with a pretreatment liquid containing the anti-C3 antibody and then dropped to a microplate, or the sample may be mixed with a solution containing the anti-C3 antibody and a detection antibody and then dropped to a microplate.

Moreover, in the case of a chemiluminescent measurement reagent, the sample may be mixed in advance with a pretreatment liquid containing the anti-C3 antibody and then mixed with the immunoassay reagent, or the anti-C3 antibody may be contained in the immunoassay reagent (for example, a solution containing an antibody or an antigen for detection and magnetic particles).

When an immunoreaction is conducted on a solid phase as on an immunochromatography or the like, an immunoreaction system (or being in an immunoreaction system) refers to a solid phase (or being on a solid phase) on which the immunoreaction of the liquid sample and the binding partner is conducted. In this case, the sample may be mixed in advance with a pretreatment liquid containing the anti-C3 antibody and then dropped to an immunochromatographic test strip. Alternatively, the anti-C3 antibody may be dried and held on a sample pad, and the anti-C3 antibody may be dissolved with the sample dropping and develop in the solid phase to thus become present in the reaction system.

In the invention, the concentration of the anti-C3 antibody may be any concentration which does not strongly affect the immunoreaction of the analyte and the specific binding partner and which can exhibit the desired effect of suppressing non-specific reaction, and one skilled in the art can appropriately set the concentration depending on the analyte and the kind of specimen. The anti-C3 antibody concentration in the immunoreaction system differs also with the reagent components of the immunoreaction system but is, as the weight of the anti-C3 antibody in 10 µL of the specimen, 0.1 to 1000 µg, preferably 1.0 to 750 µg, more preferably 2.0 to 500 µg, further preferably 3.0 to 250 µg, most preferably 5.0 to 150 µg, but the concentration is not limited to these concentration. For example, in some cases, 5.0 to 120 µg, 5.0 to 110 µg, 5.0 to 100 µg, 5.0 to 80 µg, 5.0 to 60 µg or 5.0 to 50 µg is preferable. As the lower limit of the range, 10.0 µg or more, 15.0 µg or more or 20.0 µg or more is preferable in some cases.

One kind of anti-C3 antibody may be used, or a mixture of more than one anti-C3 antibody may also be used. Moreover, a monoclonal antibody and a polyclonal antibody may be used in combination, and an anti-C3 antibody bound to an insoluble carrier may also be used. When two or more kinds of anti-C3 antibody are used in combination, the total concentration of the antibodies may be anything within the aforementioned concentration range.

When the anti-C3 antibody is contained in advance in the measurement reagent of the invention, the anti-C3 antibody can be contained in the measurement reagent in such a manner that the aforementioned concentration is achieved in the reaction system.

### (Latex Immunoagglutination Assay (LTIA))

An LTIA, which is one of the immunoassay methods of the invention, will now be explained. The methods for measuring an analyte by an LTIA can be roughly classified into two.

In the first method, latex particles carrying a specific binding partner for the analyte are reacted with the analyte to form a sandwich type immunocomplex. The analyte is then measured based on the degree of agglutination of the latex particles due to the formation of the immunocomplex.

In the second method, proteins or the like to which a plurality of analytes or their analogues (including their fragments) are immobilized are added to a reagent in advance. When these proteins, or the like, compete with the analyte in the sample, the formation of an immunocomplex between the analyte contained in the reagent and the latex particle on which the specific binding partner for the analyte is immobilized is inhibited. The analyte (antigen) is then measured from the degree of inhibition of agglutination of said latex particles due to the inhibition of the formation of immunocomplexes.

As the analyte and the specific binding partner for the analyte, any substances can be selected depending on the purpose. For example, when the analyte is an antigen, an antibody such as a polyclonal antibody and a monoclonal antibody (including a recombinant antibody and a functional fragment of an antibody) can be selected as the specific binding partner for the analyte. When the analyte is an antibody, an antigen such as native-form and recombinant antigens can be selected as the specific binding partner for the analyte. The invention of the present application can be used for both methods, and specifically, the following steps are exemplified:
(1) a step of bringing the analyte in the sample and the anti-C3 antibody into contact with each other in a solution;
(2) a step of adding latex particles carrying a specific binding partner for the analyte to the solution after the step (1);
(3) a step of optically detecting the degree of agglutination of the latex particles in the solution after the step of (2).

Here, it is intended that the step of (3) is "a step of measuring the agglutination reaction of the analyte and the latex particles during the step of (2) or after the step of (2) without any washing/separation step".

In the LTIA, the substance to be tested can be measured by optically or electrochemically observing the degree of generated agglutination. The optical observation method is a method for measuring the intensity of scattered light, the absorbance, or the intensity of transmitted light with an optical device (endpoint method, rate method or the like). The concentration (quantitative value) of the analyte contained in the sample is calculated by comparing the measured value of the absorbance or the like obtained by measuring the sample with the measured value of the absorbance or the like obtained by measuring a standard (a sample with a known concentration of the analyte). In this regard, the absorbance or the like such as transmitted light and scattered light may be measured by single wavelength measurement or dual-wavelength measurement (the difference or the ratio resulting from two wavelengths). The wavelength for the measurement is generally determined in the range of 500 nm to 900 nm.

The analyte in the sample of the invention may be measured by a hand process or using an apparatus such as a measurement apparatus. The measurement apparatus may be a general-purpose automated analyzer or a single-purpose measurement apparatus (a special purpose apparatus). The measurement is preferably conducted by a method using multiple operational steps such as a two-step method (dual-reagent method).

### (Specific Binding Partner-Carrying Latex Particles)

The specific binding partner for the analyte can be immobilized and carried on latex particles by a known method such as a physical adsorption method, a chemical bonding method or a combination thereof.

The physical adsorption method can be conducted according to a known method, for example, by mixing the specific binding partner for the analyte and latex particles in a solution such as a buffer and bringing the two into contact with each other or by bringing the specific binding partner for the analyte dissolved in a buffer or the like into contact with a carrier.

The chemical bonding method can be conducted according to a known method described in "Extra Special Issue of Clinical Pathology, No. 53, Immunoassay for Clinical Examination-Techniques and Applications-" edited by The Japanese Society of Clinical Pathology, Clinical Pathology Publication Society, issued in 1983; "New Courses in Biochemical Experiment 1, Protein IV" edited by The Japanese Biochemical Society, Tokyo Kagaku Dojin, issued in 1991 or the like, for example, by mixing and bringing the specific binding partner for the analyte and a carrier into contact with a divalent crosslinking reagent such as glutaraldehyde, carbodiimide, an imide ester and maleimide, and reacting the amino groups, the carboxyl groups, the thiol groups, the aldehyde groups, the hydroxyl groups or the like of the specific binding partner for the analyte and the carrier, with the divalent crosslinking reagent.

The synthetic polymer constituting the latex particles of the invention is not particularly limited, but examples thereof include polystyrene, a styrene-styrene sulfonate copolymer, a methacrylic acid polymer, an acrylic acid polymer, an itaconic acid polymer, a styrene-hydrophilic carboxy monomer copolymer such as a styrene-methacrylic acid copolymer, a styrene-acrylic acid copolymer and a styrene-itaconic acid copolymer and the like. Of these, a styrene-methacrylic acid copolymer, a styrene-itaconic acid copolymer, styrene and a styrene-styrene sulfonate copolymer are preferable. Styrene and a styrene-(meth)acrylic acid copolymer are particularly preferable.

The specific binding partner for a particular substance which is carried on the latex particles is preferably of more than one kind to form a sandwich. When the particular substance has more than one sites recognized by the antibody, the specific binding partner may be of one kind. For example, when the specific binding partner is a monoclonal antibody, two or more monoclonal antibodies having different recognition sites are used. Moreover, for example, when the specific binding partner is a polyclonal antibody, the polyclonal antibody may be derived from one kind of antiserum or derived from more than one kind of antiserum. Furthermore, a monoclonal antibody and a polyclonal antibody may be used in combination.

When treatment is necessary to suppress spontaneous agglutination of the latex particles, non-specific reaction or the like, the carrier may be subjected to blocking (masking) by treating by a known method such as contacting or covering of the surface of the latex particles with a protein such as bovine serum albumin (BSA), casein, gelatin, egg albumin or salts thereof, a surfactant, defatted milk powder or the like.

### (Immunoassay Reagent)

The immunoreaction of the immunoassay method of the invention is conducted using an immunoassay reagent, and the reagent is characterized by containing the anti-C3 antibody in addition to the main reaction components. The main reaction components include the specific binding partner for the analyte (except for the anti-C3 antibody) as well as an insoluble carrier such as particles for immunoassay, an immunochromatographic test strip and a microplate and the like.

The immunoassay reagent of the invention may contain a buffer, a protein, a peptide, an amino acid, a nucleic acid, a lipid, a phospholipid, a saccharide, an inorganic salt, a polymer compound, a surfactant, another agent for suppressing non-specific reaction, a preservative or the like as long as the effect of suppressing non-specific reaction is not inhibited. As a component for buffering or adjusting the pH, the ionic strength, the osmotic pressure or the like of the sample, for example, the reagent may contain a buffer such as acetic acid, citric acid, phosphoric acid, Tris, glycine, boric acid, carbonic acid, phthalic acid, succinic acid, maleic acid, imidazole, and Good's buffers as well as sodium salt, potassium salt, calcium salt or the like thereof. Moreover, the reagent may contain a polymer such as polyvinyl pyrrolidone and a phospholipid polymer as a component for enhancing the agglutination formation.

The anti-C3 antibody concentration in the constituting reagent may be in such a manner that the concentration can be adjusted to the concentration in the immunoreaction system when the reagent and the sample are mixed, namely at the time of measurement, and differs with the reagent type.

### (Reagent Kit)

The reagent kit of the invention is characterized by containing at least an anti-C3 antibody in the kit components. The kit components include a sample diluent, a sample extraction solution and the like in addition to a reagent involving the immunoassay, but in the invention, the anti-C3 antibody may be added to any one thereof or to two or more thereof. The kit components may also include a user guide, a sample collection tool (a collection pipette, a syringe, a cotton swab, a filter and the like).

The reagent components employing immunoassay methods will be each explained.

### <Latex Immunoagglutination Measurement Method>

A reagent (LTIA reagent) for a latex immunoagglutination measurement method as the immunoassay method is shown as an example.
(1) A first reagent containing an anti-C3 antibody.
(2) A second reagent containing latex particles carrying a specific binding partner for the analyte.

The first reagent typically contains a buffer, and the anti-C3 antibody concentration in the buffer may be any form such that the concentration can be adjusted to the preferable anti-C3 antibody concentration when the reagent and the sample are mixed, namely at the time of measurement, and differs with the reagent type. The anti-C3 antibody may be contained in the first reagent as well as in the second reagent.

In the LTIA reagent, the concentration of the anti-C3 antibody contained in a general first reagent is 1 to 2000 µg/mL, preferably 5 to 1500 µg/mL, more preferably 10 to 1000 µg/mL, further preferably 15 to 500 µg/mL, most preferably 25 to 500 µg/mL but is not limited to the concentration.

When a specimen diluent, a pretreatment liquid or the like is added to the specimen before subjecting the specimen to an LTIA, the anti-C3 antibody may be contained in the pretreatment liquid, and the anti-C3 antibody concentration in the pretreatment liquid is 1 to 2000 µg/mL, preferably 5 to 1500 µg/mL, more preferably 10 to 1000 µg/mL, further preferably 15 to 500 µg/mL, most preferably 25 to 500 µg/mL but is not limited to the concentration.

The anti-C3b antibody, the anti-iC3b antibody, the anti-C3dg antibody and the anti-C3d antibody can also be used in a similar concentration range to that of the anti-C3 antibody mentioned above.

As the immunoassay particles used for the invention, known particles which can carry the specific binding partner for the analyte can be used in addition to the latex particles. For example, inorganic particles of metal colloid, silica, carbon or the like can also be used as the immunoassay particles of the invention.

The size of the immunoassay particles can be appropriately determined in the range of 0.05 to 1 µm in such a manner that desired measurement sensitivity, a desired measurement range or the like can be obtained considering the used optical measurement method (for example, a turbidimetry for measuring transmitted light, a nephelometry for measuring scattered light or the like). An average particle diameter of 0.1 to 0.4 µm is generally used in optical measurement in an automated analyzer, but the size is not limited thereto.

### <ELISA>

An ELISA is a method which uses a combination of various types of antigen-antibody reaction and in which an enzyme-labeled antigen or antibody is incorporated into the reaction system in the end to detect the enzymatic activity. To detect the enzymatic activity, a substrate having an absorption spectrum that changes with the reaction is used, and there are a direct assay, an indirect assay, a sandwich assay, a competitive assay and the like depending on the combination of the types of antigen-antibody reaction.

A reagent for a sandwich ELISA as the immunoassay method of the invention is shown as an example:
(a) an insoluble carrier on which an antibody reacting with the analyte is immobilized;
(b) an antibody which is labeled with a labeling substance and which reacts with the analyte.

The insoluble carrier of (a) is preferably a plate, and the labeling substance can be appropriately selected and used. The antibody immobilized on the insoluble carrier captures the analyte in a solution containing the sample and forms a complex on the insoluble carrier. The labeling substance-labeled antibody binds to the captured analyte and forms a sandwich with the complex. By measuring the labeling substance amount by a method suitable for the labeling substance, the analyte in the sample can be measured. As specific methods, such as the method for immobilizing the antibody on the insoluble carrier and the method for binding the antibody and the labeling substance, methods known to one skilled in the art can be used without any particular restriction.

In the ELISA, the anti-C3 antibody used for the invention can be caused to be present in the immunoreaction system, for example, by adding to a sample diluent, a pretreatment liquid or the like or adding to a solution for conducting the antigen-antibody reaction.

### <Immunochromatography>

The reagent components (test strip components) of immunochromatography as the immunoassay method of the invention are explained.

### Immunochromatographic Test Strip;

When an antibody is used as the specific binding partner, the test strip has "1. a sample application part", "2. a part holding a labeling antibody (labeling antibody-holding part)" and "3. a part on which an antibody for capturing the complex formed by the labeling antibody and an antibody against the analyte is immobilized (capturing antibody part)" on an insoluble carrier sheet such as a porous membrane in this order in the direction of the development of the solution containing the sample.

The immunochromatography includes at least the aforementioned test strip. When a predetermined amount of a sample containing the analyte is added to the sample application part, the sample enters the label-holding part through a capillary phenomenon, and the analyte and the labeling antibody bind to each other and form a complex. The complex develops in the membrane and is captured by the antibody immobilized on the membrane (capturing antibody) when the complex enters the capturing antibody part, and a capturing antibody-analyte-labeling antibody complex is formed. By detecting the label by a method (for example, an agglutination image when the label can be visualized as in colloidal gold or the like or chromogenic reaction through addition of a substrate in the case of an enzyme), the analyte can be detected.

In the immunochromatography, the anti-C3 antibody used for the invention can be caused to be present in the reaction system, for example, by adding to a sample diluent, a pretreatment liquid or the like or adding in the sample application part or the label-holding part, then drying and holding.

### <Chemiluminescent Measurement>

In the method, after reacting antigen- or antibody-bound magnetic particles and the analyte to form a complex, unreacted substances are removed utilizing a magnet. After further adding a reagent containing a labeling antibody and removing unreacted substances utilizing a magnet, a luminescent reagent is added, and the luminous intensity is measured. An assay using an enzyme as the label is called a chemiluminescent enzyme immunoassay (CLEIA). An assay in which the luminous intensity is measured by electrochemical reaction using a metal complex such as a ruthenium-pyridine complex as the label is called an electro chemiluminescence immunoassay (ECLIA). An assay using a chemiluminescent substance as the label is called a chemiluminescent immunoassay (CLIA).

A reagent for a CLEIA as the immunoassay method of the invention is shown as an example.
(a) Magnetic particles on which an antibody (or an antigen) which reacts with the analyte is immobilized.
(b) An antibody (or an antigen) which is labeled with an enzyme and which reacts with the analyte.
(c) A luminescent reagent.

The antibody immobilized on the magnetic particles captures the analyte in a solution containing the sample and forms a complex. The antibody labeled with the enzyme labeling substance binds to the captured analyte and forms a sandwich with the complex. By reacting the enzyme labeling substance and the luminescent reagent and measuring the luminous intensity, the analyte in the sample can be measured.

In the CLEIA, the anti-C3 antibody used for the invention can be caused to be present in the immunoreaction system, for example, by adding to a sample diluent, a pretreatment liquid or the like or adding to the solution in which the antigen-antibody reaction is conducted.

### (Specific Binding Partner)

In the invention, the specific binding partner for the analyte may be a protein other than the anti-C3 antibody, a peptide, an amino acid, a lipid, a saccharide, a nucleic acid, a hapten or the like. Although whether the molecular weight is high or low and the origin thereof, such as natural or synthetic, are not particularly restricted, the specific binding partner may be an antibody or an antigen which can be used for an immunoassay.

The antibody may be a polyclonal antibody or a monoclonal antibody. A monoclonal antibody is more preferable.

In the invention, as the antibody, functional fragments of an antibody having antigen-antibody reactivity can also be used in addition to the whole antibody molecules. The antibody may be an antibody obtained through an immunization step of a general animal (a mouse, a goat, a sheep or the like) or an antibody in which the amino acid sequence has been changed to a sequence of an animal species that is different from the animal immunized with the immunogen (the substance-to-be-measured / analyte) using genetic recombination technique or the like (a chimeric antibody, a humanized antibody, a completely humanized antibody or the like). The functional fragments of the antibody include F(ab')₂ and Fab', which are fragments having antigen-antibody reactivity, as well as a single-chain antibody (scFv), a VHH (variable domain of heavy chain of heavy chain antibody) antibody, a IgNAR (new antigen receptor) antibody and the like. The functional fragments of the antibody can be produced by treating an antibody obtained as described above with a protease (for example, pepsin, papain or the like).

### (Sample)

Examples of the sample containing the analyte of the invention include blood, serum, plasma, a culture supernatant, urine, spinal fluid, saliva, sweat, ascites, an extract of cells or a tissue and the like of a human or an animal.

The samples in the invention include a sample subjected to pretreatment such as dilution and purification in addition to a sample itself obtained from a living body.

### (Substance-to-be-measured / Analyte)

The immunoassay reagent of the invention can employ various substances contained in the sample as the analyte. The analyte is a substance which does not react with the anti-C3 antibody used for suppressing non-specific reaction. The analyte may be a protein, a peptide, an amino acid, a lipid, a saccharide, a nucleic acid, a hapten or the like but is not particularly restricted as long as the substance can be measured theoretically. Examples thereof include CRP (C-reactive protein), Lp(a), MMP3 (matrix metalloproteinase-3), an anti-phospholipid antibody, collagen IV, PSA, BNP (brain natriuretic peptide), insulin, albumin, cystatin C, RF (rheumatoid factor), KL-6, procalcitonin, FDP, D-dimer, SF (soluble fibrin), TAT (thrombin-antithrombin III complex), PAI-1, phenytoin, phenobarbital, carbamazepine, valproic acid, theophylline, TARC (thymus and activation-regulated chemokine), sIL-2R (soluble interleukin-2 receptor) and the like.

### (Method for Suppressing Non-specific Reaction)

Some components contained in a biological sample often cause agglutination which should have not caused (a positive measurement error) or do not cause agglutination which should have caused (a negative measurement error) of immunoassay particles on which a specific binding partner for the analyte is immobilized. These are called non-specific reaction and are known to cause various measurement errors.

In the invention, to suppress non-specific reaction refers to act on a factor which causes the non-specific reaction (a non-specific factor) in the biological sample and to suppress the influence of reaction other than the immunoreaction on the measurement. The invention can suppress non-specific reaction by conducting the immunoreaction in the presence of an anti-C3 antibody.

### (Agent for Suppressing Non-specific Reaction)

The agent for suppressing non-specific reaction of the invention should contain a substance which can suppress non-specific reaction derived from the sample in an immunoassay method and contains at least an anti-C3 antibody as an active ingredient. For the agent for suppressing non-specific reaction of the invention, the components including the anti-C3 antibody in the reagent components can be used as they are or without any modifications or changes. The agent for suppressing non-specific reaction of the invention may contain a buffer, a protein, a peptide, an amino acid, a nucleic acid, a lipid, a phospholipid, a saccharide, an inorganic salt, a polymer compound, a surfactant, another agent for suppressing non-specific reaction, a preservative or the like as long as the effect of suppressing non-specific reaction is not inhibited.

The invention is explained in detail below referring to Examples, but the invention should not be limited to the Examples below.

### EXAMPLES

### [Example I: Suppression of Non-specific Reaction in LTIA: Measurement of TARC]

A test for measuring the TARC concentrations of samples using reagents by an LTIA with the addition of an anti-C3d polyclonal antibody of the invention of the present application and another agent for suppressing non-specific reaction is shown below. The samples used were samples (control specimens: specimens 1 to 3) showing close values to the measured values by a chemiluminescent enzyme immunoassay (CLEIA) (Comparative Example 1) and samples (divergent specimens: specimens 4 to 10) which exhibit non-specific reaction and which have values largely different from the measured values of Comparative Example 1

### [Comparative Example (sometimes abbreviated as "Cmp. Ex.") 1] (Measurement by CLEIA)

### 1. Measurement Method

### 1-1. Measurement Reagent

HISCL (registered trademark) TARC (Sysmex Corporation)

### 1-2. Samples

Physiological saline (Otsuka normal saline) and specimens (serum) 1-10

### 1-3. Measurement Procedures

Measurement was made using HISCL (registered trademark) -5000 (Sysmex Corporation) according to the documents attached to the reagent.

### 2. Measurement Results

The measurement results are shown in Table 1.

The CLEIA shown in Comparative Example 1 includes B/F separation operation and has a washing step. Accordingly, the CLEIAis a measurement method which is not easily affected by sample-derived non-specific reaction and was thus used as a Comparative Example.

### [Comparative Example 2] (Measurement by LTIA: Without Addition of Agents for Suppressing Non-specific Reaction)

### 1. Measurement Method

### 1-1. Measurement Reagent

An LTIA reagent composed of the first reagent and the second reagent shown below was prepared.
(1) First Reagent
   100 mM MOPS-NaOH (pH7.5)
   500 mM NaCl
   0.5% BSA
(2) Second Reagent
   Anti-human TARC monoclonal antibody-sensitized latex (2 types)
   5 mM MOPS-NaOH (pH7.0)

Here, the anti-human TARC monoclonal antibodies were acquired using a commercial TARC antigen by a method known to one skilled in the art. Commercial TARC antigens include CCL17, thymus and activation regulated chemokine (Shenandoah Biotechnology, Inc.), CCL17/TARC, Human (LifeSpan Biosscience, Inc.) and Human TARC (CCL17) (Abeomics, Inc.). Furthermore, a combination of monoclonal antibodies which could be used for a sandwich assay of a TARC antigen was selected by a method known to one skilled in the art. The anti-human TARC monoclonal antibody-sensitized latex was prepared referring to the method described in JP-A-2017-181377.

### 1-2. Samples

Same as the samples of Comparative Example 1.

### 1-3. Measurement Procedures

The first reagent and the second reagent were combined, and the TARC concentrations of the samples were measured using Hitachi autoanalyzer 3500. Specifically, after adding 120 µL of the first reagent to 2.4 µL of a sample and incubating at 37°C for five minutes, 40 µL of the second reagent was added and stirred. The change in absorbance due to agglutination formation was measured for the following five minutes at a main wavelength of 570 nm and a sub-wavelength of 800 nm, and the measured value was calculated by applying the change in absorbance to a calibration curve obtained by measuring a standard with a known concentration.

### 2. Measurement Results

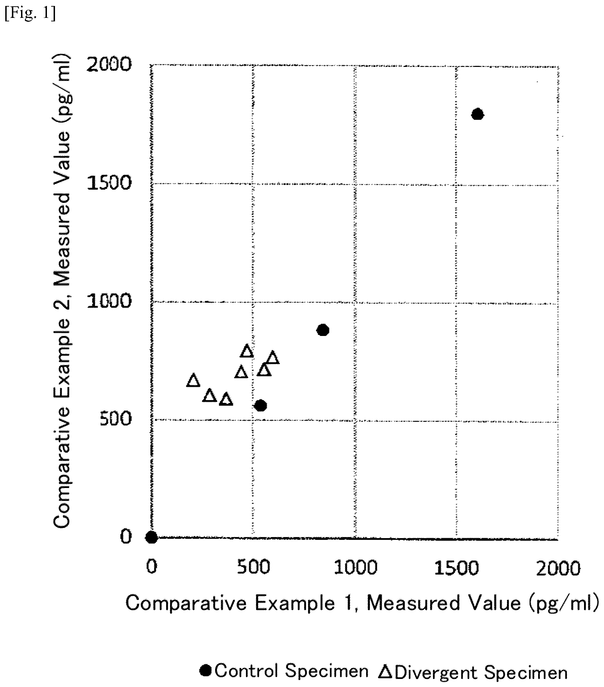

The measurement results are shown in Table 1. Moreover, the correlation of the measured values of Comparative Example 2 to the measured values of Comparative Example 1 is shown in Fig. 1.

### [Comparative Example 3] (Measurement by LTIA: Addition of HBR-1)

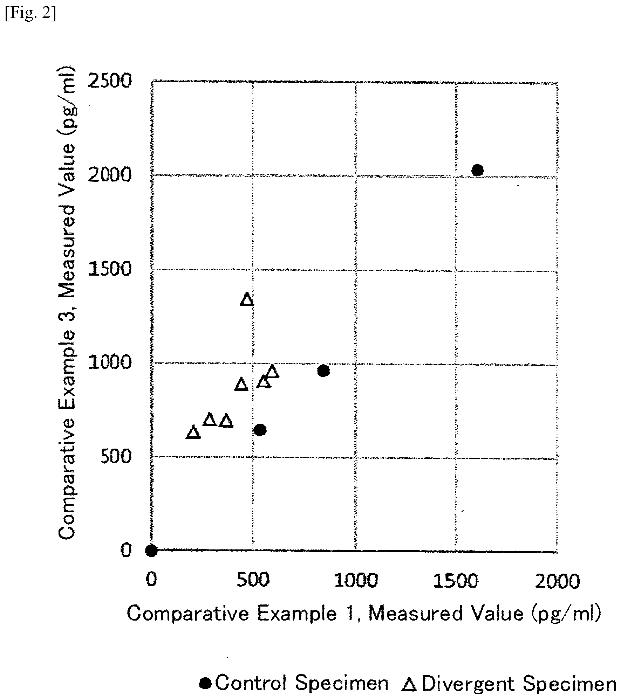

Measurement was made by the same method as that in Comparative Example 2 except for adding a commercial agent for suppressing non-specific reaction, HBR-1(SCANTIBODIES LABORATORY, INC.) to the first reagent shown in Comparative Example 2 at 100 µg/mL. The measurement results are shown in Table 1. Moreover, the correlation of the measured values of Comparative Example 3 to the measured values of Comparative Example 1 is shown in Fig. 2.

### [Example 1] (Measurement by LTIA: Addition of Anti-C3d Polyclonal Antibody)

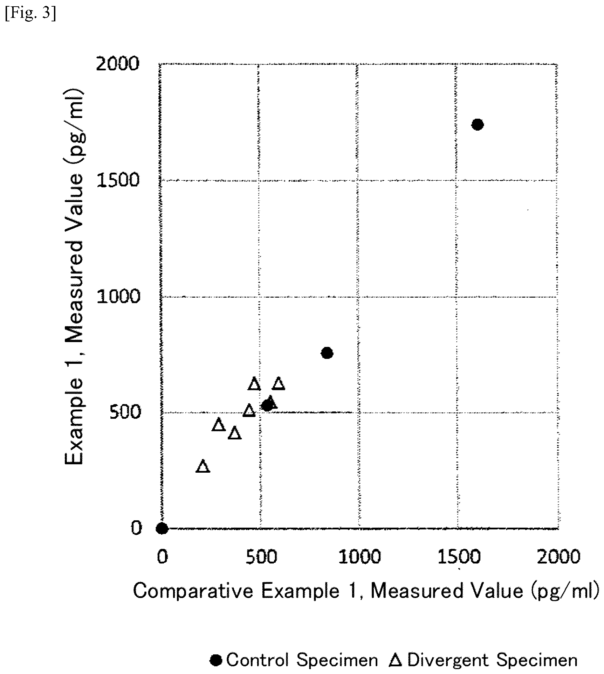

Measurement was made by the same method as that in Comparative Example 2 except for adding an anti-C3d polyclonal antibody (Bio-Rad) to the first reagent shown in Comparative Example 2 at 100 µg/mL. The measurement results are shown in Table 1. Moreover, the correlation of the measured values of Example 1 to the measured values of Comparative Example 1 is shown in Fig. 3.

**[Table 1]**

| | Measured TARC Values (pg/mL) of Samples | | | | |
|---|---|---|---|---|---|
| | Specimen No. | Measured TARC Value (pg/mL) | | | |
| | | Cmp. Ex. 1 CLEIA | Cmp. Ex. 2 LTIA | Cmp. Ex. 3 LTIA | Example 1 LTIA |
| | | - | Without Additives | Addition of 100 *µ* g/mL of HBR-1 | Addition of 100 *µ* g/mL of Anti-C3d Polyclonal Antibody |
| Control Specimen | Physiological Saline | 0 | 0 | 0 | 0 |
| | 1 | 537 | 561 | 645 | 531 |
| | 2 | 843 | 884 | 963 | 759 |
| | 3 | 1604 | 1800 | 2037 | 1741 |
| Divergent Specimen | 4 | 205 | 669 | 634 | 271 |
| | 5 | 284 | 606 | 700 | 450 |
| | 6 | 366 | 591 | 696 | 415 |
| | 7 | 441 | 705 | 891 | 512 |
| | 8 | 468 | 796 | 1347 | 626 |
| | 9 | 553 | 717 | 906 | 545 |
| | 10 | 594 | 768 | 960 | 628 |

### (Results and Discussion)

From the results of Comparative Examples 1 to 3, Example 1 and Reference Example 1, the effects of the invention of the present application were discussed.
(1) The measurement results of the control specimens (specimen Nos. 1 to 3) were examined.

The measured values of the control specimens by the CLEIA (Comparative Example 1) and by the LTIA without the addition of the agents for suppressing non-specific reaction (Comparative Example 2) were approximately equivalent. Moreover, the measured values by the LTIA with the addition of the anti-C3d polyclonal antibody of the invention of the present application (Example 1) were also approximately equivalent to those of Comparative Examples 1 and 2. Accordingly, it was found that the addition of the anti-C3d polyclonal antibody did not affect the measured values of the specimens exhibiting no non-specific reaction. Here, in Comparative Example 3, the measured value of specimen 3 differed from that of Comparative Example 1. It was believed that undesirable reaction occurs when HBR-1 is added.

(2) The measurement results of the divergent specimens (specimen Nos. 4 to 10) were examined.

While the measured value of specimen No. 4 by the CLEIA (Comparative Example 1) was 205 pg/mL, the measured value thereof by the LTIA without the addition of the agents for suppressing non-specific reaction (Comparative Example 2) was 669 pg/mL, which were largely different measured values. Furthermore, the measured value by the LTIA with the addition of HBR-1 (Comparative Example 3) was 634 pg/mL, which was largely different from that of Comparative Example 1. On the other hand, the measured value by the LTIA with the addition of the anti-C3d polyclonal antibody of the invention of the present application (Example 1) was 271 pg/mL, showing a tendency towards a value closer to that of Comparative Example 1. Similar results were obtained also for specimens 5 to 10.

From the above results, by the presence of the anti-C3d antibody of the invention of the present application in the immunoreaction system in an immunoassay, sample-derived non-specific reaction could be suppressed. That the invention could suppress non-specific reaction which could not be suppressed even with a commercial agent for suppressing non-specific reaction, HBR-1, was an unexpected result.

Here, although the divergent specimens used in the test were specimens containing RF at concentrations of a wide range, no relevance was found between the amount of RF and the divergence of the measured values (data not shown). Accordingly, it was suggested that the effect of the anti-C3d antibody of the invention of the present application of suppressing non-specific reaction was not likely to be affected by the amount of RF.

### [Example II: Suppression of Non-specific Reaction in LTIA: Measurement of sIL-2R]

A test for measuring the sIL-2R concentrations of samples using reagents by an LTIA with the addition of an anti-C3d polyclonal antibody of the invention of the present application and another agent for suppressing non-specific reaction is shown below. The samples used were samples (control specimens: specimens 11 to 15) showing close values to the measured values by a chemiluminescent enzyme immunoassay (CLEIA) (Comparative Example 4) and samples (divergent specimens: specimens 16 and 17) which exhibit non-specific reaction and which have values largely different from the measured values of Comparative Example 4.

### [Comparative Example 4] (Measurement by CLEIA)

### 1. Measurement Method

### 1-1. Measurement Reagent

Lumipulse Presto (registered trademark) IL-2R (FUJIREBIO Inc.)

### 1-2. Samples

Specimens (serum) 11-17

### 1-3. Measurement Procedures

Measurement was made using Lumipulse (registered trademark) -L2400 (FUJIREBIO Inc.) according to the documents attached to the reagent.

### 2. Measurement Results

The measurement results are shown in Table 2.

The CLEIA shown in Comparative Example 4 includes B/F separation operation and has a washing step. Accordingly, the CLEIAis a measurement method which is not easily affected by sample-derived non-specific reaction and was thus used as a Comparative Example.

### [Comparative Example 5] (Measurement by LTIA: Without Addition of Agents for Suppressing Non-specific Reaction)

### 1. Measurement Method

### 1-1. Measurement Reagents

A first reagent and a second reagent were prepared according to the method described in JP-A-2017-181377.

### 1-2. Samples

Same as the samples of Comparative Example 4.

### 1-3. Measurement Procedures

The first reagent and the second reagent were combined, and the sIL-2R concentrations of the samples were measured using Hitachi type 7180 autoanalyzer. Specifically, after adding 120 µL of the first reagent to 5.6 µL of a sample and incubating at 37°C for five minutes, 40 µL of the second reagent was added and stirred. The change in absorbance due to agglutination formation was measured for the following five minutes at a main wavelength of 570 nm and a sub-wavelength of 800 nm, and the measured value was calculated by applying the change in absorbance to a calibration curve obtained by measuring a standard with a known concentration.

### 2. Measurement Results

The measurement results are shown in Table 2.

### [Comparative Example 6] (Measurement by LTIA: Addition of HBR-1)

Measurement was made by the same method as that in Comparative Example 5 except for adding HBR-1 (SCANTIBODIES LABORATORY, INC.) to the first reagent shown in Comparative Example 5 at 100 µg/mL. The measurement results are shown in Table 2.

### [Example 2] (Measurement by LTIA: Addition of Anti-C3d Polyclonal Antibody)

Measurement was made by the same method as that in Comparative Example 5 except for adding an anti-C3d polyclonal antibody (Bio-Rad) to the first reagent shown in Comparative Example 5 at 100 µg/mL. The measurement results are shown in Table 2.

**[Table 2]**

| | Measured sIL-2R Values (U/mL) of Samples | | | | |
|---|---|---|---|---|---|
| | Specimen No. | Measured sIL-2R Value (U/mL) | | | |
| | | Cmp. Ex. 4 CLEIA | Cmp. Ex. 5 LTIA | Cmp. Ex. 6 LTIA | Example 2 LTIA |
| | | - | Without Additives | Addition of 100 *µ* g/mL of HBR-1 | Addition of 100 *µ* g/mL of Anti-C3d Polyclonal Antibody |
| Control Specimen | 11 | 405 | 444 | 488 | 446 |
| | 12 | 1400 | 1441 | 1501 | 1484 |
| | 13 | 449 | 494 | 514 | 485 |
| | 14 | 611 | 710 | 728 | 704 |
| | 15 | 1050 | 1057 | 1099 | 1089 |
| Divergent Specimen | 16 | 346 | 1401 | 1216 | 409 |
| | 17 | 171 | 500 | 392 | 297 |

### (Results and Discussion)

From the results of Comparative Examples 4 to 6 and Example 2, the effects of the invention of the present application were discussed.
(1) The measurement results of the control specimens (specimen Nos. 11 to 15) were examined.
   The measured values of the control specimens by the CLEIA (Comparative Example 4) and by the LTIA without the addition of the agents for suppressing non-specific reaction (Comparative Example 5) were approximately equivalent. Moreover, the measured values by the LTIA with the addition of the anti-C3d polyclonal antibody of the invention of the present application (Example 2) were also approximately equivalent to those of Comparative Examples 4 and 5. Accordingly, it was found that the addition of the anti-C3d polyclonal antibody did not affect the measured values of the specimens exhibiting no non-specific reaction.
(2) The measurement results of the divergent specimens (specimen Nos. 16 and 17) were examined.

While the measured value of specimen No. 16 by the CLEIA (Comparative Example 4) was 346 U/mL, the measured value thereof by the LTIA without the addition of the agents for suppressing non-specific reaction (Comparative Example 5) was 1401 U/mL, which were largely different measured values. Furthermore, the measured value by the LTIA with the addition of HBR-1 (Comparative Example 5) was 1216 U/mL, which was largely different from that of Comparative Example 4.

On the other hand, the measured value by the LTIA with the addition of the anti-C3d polyclonal antibody of the invention of the present application (Example 2) was 409 U/mL, showing a tendency towards a value closer to that of Comparative Example 4. Similar results were obtained also for specimen 17.

From the above results, by the presence of the anti-C3d antibody of the invention of the present application in the immunoreaction system in an immunoassay, sample-derived non-specific reaction could be suppressed. Because the invention could suppress non-specific reaction which could not be suppressed even with a commercial agent for suppressing non-specific reaction, HBR-1, the effect of the anti-C3d antibody of the invention of suppressing non-specific reaction was an unexpected result.

### [Example III: Suppression of Non-specific Reaction in ELISA: Measurement of sIL-2R]

A test for measuring the sIL-2R concentrations of samples using reagents by an ELISA with the addition of an anti-C3d polyclonal antibody of the invention of the present application and another agent for suppressing non-specific reaction is shown below. The samples used were samples (control specimens: specimens 18 to 22) showing close values to the measured values by a chemiluminescent enzyme immunoassay (CLEIA) (Comparative Example 7) and a sample (a divergent specimen: specimen 23) which exhibits non-specific reaction and which has a value largely different from the measured values of Comparative Example 7.

### [Comparative Example 7] (Measurement by CLEIA)

### 1. Measurement Method

### 1-1. Measurement Reagent

Lumipulse Presto (registered trademark) IL-2R (FUJIREBIO Inc.)

### 1-2. Samples

Serum specimens 18-23

### 1-3. Measurement Procedures

Measurement was made using Lumipulse (registered trademark) L2400 (FUJIREBIO Inc.) according to the documents attached to the measurement reagent.

### 2. Measurement Results

The measurement results are shown in Table 3.

### [Comparative Example 8] (Measurement by ELISA: Without Addition of Agents for Suppressing Non-specific Reaction)

### 1. Measurement Method

### 1-1. Samples

Same as the samples of Comparative Example 7.

### 1-2. Measurement Procedures

The same antibody as the antibody used in Comparative Example 5 was used.

An anti-sIL-2R monoclonal antibody was dissolved in a 20 mM phosphate buffer containing 150 mM sodium chloride (pH7.2; called PBS below) at 10 µg/mL, and 100 µL of the solution was dispensed to the wells of a 96-well microplate. The plate was left still at 4°C overnight.

After washing the wells three times with 400 µL of PBS containing 0.05% Tween (registered trademark) 20 (called PBST below), 200 µL of PBST containing 1% bovine serum albumin (called BSA-PBST below) was added, and the wells were blocked at room temperature for an hour. The plate was used as an ELISA plate.

After washing the wells of the ELISA plate three times with 400 µL of PBST, 100 µL of the samples which were diluted 40-fold with a specimen diluent (BSA-PBST) were added to the wells, and the plate was left still at room temperature for an hour. After washing the wells three times with 400 µL of PBST, 100 µL of a biotin-labeled anti-sIL-2R monoclonal antibody which was diluted to 0.50 µg/mL with BSA-PBST was dispensed to the wells, and the plate was left still at room temperature for an hour. After washing the wells three times with 400 µL of PBST, 100 µL of HRP-labeled streptavidin (ThermoFishier Scientific) which was diluted to 0.20 µg/mL with BSA-PBST was dispensed to the wells, and the plate was left still at room temperature for 30 minutes. After washing the wells three times with 400 µL of PBST, 50 µL of a citrate buffer (pH5.0) containing 0.2% o-phenylenediamine and 0.02% hydrogen peroxide was added, and the plate was left at room temperature for 10 minutes. Then, the enzymatic reaction was stopped by adding 50 µL of 4.5N sulfuric acid, and the absorbances at a wavelength of 492 nm were measured.

A standard with a known concentration was used as a calibrator, and the sIL-2R values in the samples to be tested were calculated. The measurement results are shown in Table 3.

### [Comparative Example 9] (Measurement by ELISA: Addition of HBR-1)

Measurement was made by the same method as that in Comparative Example 8 except for adding HBR-1 (SCANTIBODIES LABORATORY, INC.) to the specimen diluent (BSA-PBST) shown in Comparative Example 8 at 100 µg/mL. The measurement results are shown in Table 3.

### [Example 3] (Measurement by ELISA: Addition of Anti-C3d Polyclonal Antibody)

Measurement was made by the same method as that in Comparative Example 8 except for adding an anti-C3d polyclonal antibody (Bio-Rad) to the specimen diluent (BSA-PBST) shown in Comparative Example 8 at 100 µg/mL. The measurement results are shown in Table 3.

**[Table 3]**

| | Measured sIL-2R Values (U/mL) of Samples | | | | |
|---|---|---|---|---|---|
| | Specimen No. | Measured IL-2R Value (U/mL) | | | |
| | | Cmp. Ex. 7 CLEIA | Cmp. Ex. 8 ELISA | Cmp. Ex. 9 ELISA | Example 3 ELISA |
| | | - | Without Additives | Addition of 100 *µ* g/mL of HBR-1 | Addition of 100 *µ* g/mL of Anti-C3d Polyclonal Antibody |
| Control Specimen | 18 | 379 | 389 | 372 | 379 |
| | 19 | 568 | 557 | 622 | 579 |
| | 20 | 1021 | 985 | 1036 | 953 |
| | 21 | 2009 | 2096 | 2056 | 2101 |
| | 22 | 3782 | 3529 | 3674 | 3623 |
| Divergent Specimen | 23 | 1534 | 2132 | 2155 | 1338 |

### (Results and Discussion)

From the results of Comparative Examples 7 to 9 and Example 3, the effects of the invention of the present application were discussed.
(1) The measurement results of the control specimens (specimen Nos. 18 to 22) were examined.
   The measured values of the control specimens by the CLEIA (Comparative Example 7) and by the ELISA without the addition of the agents for suppressing non-specific reaction (Comparative Example 8) were approximately equivalent. Moreover, the measured values by the LTIA with the addition of the anti-C3d polyclonal antibody of the invention of the present application (Example 3) were also approximately equivalent to those of Comparative Examples 7 and 8. Accordingly, it was found that the addition of the anti-C3d polyclonal antibody did not affect the measured values of the specimens exhibiting no non-specific reaction.
(2) The measurement results of the divergent specimen (specimen No. 23) were examined.

While the measured value of specimen No. 23 by the CLEIA (Comparative Example 7) was 1534 U/mL, the measured value thereof by the ELISA without the addition of the agents for suppressing non-specific reaction (Comparative Example 8) was 2132 U/mL, which were largely different measured values. Furthermore, the measured value by the ELISA with the addition of HBR-1 (Comparative Example 9) was 2155 U/mL, which was largely different from that of Comparative Example 7.

On the other hand, the measured value by the ELISA with the addition of the anti-C3d polyclonal antibody of the invention of the present application (Example 3) was 1338 U/mL, showing a tendency towards a value closer to that of Comparative Example 4. As a result, it was found that some non-specific reaction caused in Comparative Examples 8 and 9 could be suppressed by adding the anti-C3d polyclonal antibody.

From the above results, by the presence of the anti-C3d antibody of the invention of the present application in the immunoreaction system in an immunoassay, non-specific reaction could be suppressed. Because the invention could suppress non-specific reaction which could not be suppressed even with a commercial agent for suppressing non-specific reaction, HBR-1, the effect of the anti-C3d antibody of the invention of suppressing non-specific reaction was an unexpected result. Such an effect of suppressing non-specific reaction was found to be exhibited not only in the homogeneous assay system of Examples I and II but also in the heterogeneous assay system of Example III.

### [Example IV: Suppression of Non-specific Reaction in LTIA: Measurement of sIL-2R]

A test for measuring the sIL-2R concentrations of samples using reagents by an LTIA with the addition of an anti-C3d polyclonal antibody of the invention of the present application and another agent for suppressing non-specific reaction is shown below. The samples used were samples (control specimens: specimens 12, 24 and 25) showing close values to the measured values by a chemiluminescent enzyme immunoassay (CLEIA) (Comparative Example 10) and samples (divergent specimens: specimens 16 and 17) which exhibit non-specific reaction and which have values largely different from the measured values of Comparative Example 10.

### [Comparative Example 10] (Measurement by CLEIA)

### 1. Measurement Method

### 1-1. Measurement Reagent

Lumipulse Presto (registered trademark) IL-2R (FUJIREBIO Inc.)

### 1-2. Samples

Specimens (serum) 12, 16, 17, 24 and 25

### 1-3. Measurement Procedures

Measurement was made using Lumipulse (registered trademark) -L2400 (FUJIREBIO Inc.) according to the documents attached to the reagent.

### 2. Measurement Results

The measurement results are shown in Table 4.

### [Comparative Example 11] (Measurement by LTIA: Without Addition of Agents for Suppressing Non-specific Reaction)

### 1. Measurement Method

### 1-1. Measurement Reagents

The first reagent and the second reagent prepared in Comparative Example 5 were used.

### 1-2. Samples

Same as the samples of Comparative Example 10.

### 1-3. Measurement Procedures

Measurement was made by the same method as that in Comparative Example 5.

### 2. Measurement Results

The measurement results are shown in Table 4.

### [Comparative Example 12] (Measurement by LTIA: Addition of HBR-1)

The same reagents as those of Comparative Example 6 were used. The measurement results are shown in Table 4.

### [Example 4] (Measurement by LTIA: Addition of Anti-C3d Polyclonal Antibody)

Measurement was made by the same method as that in Comparative Example 5 except for adding an anti-C3d polyclonal antibody (Bio-Rad) to the first reagent shown in Comparative Example 5 at 25 µg/mL (Example 4-1), 50 µg/mL (Example 4-2), 100 µg/mL (Example 4-3), 200 µg/mL (Example 4-4) and 500 µg/mL (Example 4-5). The measurement results are shown in Table 4.

**[Table 4]**

| | Measured sIL-2R Values (U/mL) of Samples | | | | | | | | |
|---|---|---|---|---|---|---|---|---|---|
| | Specimen No. | Measured IL-2R Value (U/mL) | | | | | | | |
| | | Cmp. Ex. 10 CLEIA | Cmp. Ex. 11 LTIA | Cmp. Ex. 12 LTIA | Example 4-1 LTIA | Example 4-2 LTIA | Example 4-3 LTIA | Example 4-4 LTIA | Example 4-5 LTIA |
| | | - | Without Additives | Addition of 100 *µ* g/mL of HBR-1 | Addition of 25 *µ* g /mL of Anti-C3d Polyclonal Antibody | Addition of 50 *µ* g/mL of Anti -C3d Polyclonal Antibody | Addition of 100 *µ* g/mL of Anti -C3d Polyclonal Antibody | Addition of 200 *µ* g/mL of Anti -C3d Polyclonal Antibody | Addition of 500 *µ* g/mL of Anti -C3d Polyclonal Antibody |
| Control Specimen | 12 | 1400 | 1378 | 1421 | 1356 | 1414 | 1375 | 1408 | 1412 |
| | 24 | 450 | 513 | 528 | 526 | 542 | 507 | 536 | 558 |
| | 25 | 718 | 756 | 782 | 753 | 758 | 773 | 758 | 794 |
| Divergent Specimen | 16 | 346 | 1419 | 1216 | 352 | 395 | - | - | 374 |
| | 17 | 171 | 474 | 349 | 285 | 257 | 285 | 274 | 243 |

| | | | | | | | | | |
|---|---|---|---|---|---|---|---|---|---|
| -;Not Measured | | | | | | | | | |

### (Results and Discussion)

From the results of Comparative Examples 10 to 12 and Examples 4-1 to 4-5, the effects of the invention of the present application were discussed.
(1) The measurement results of the control specimens (specimen Nos. 12, 24 and 25) were examined.
   The measured values of the control specimens by the CLEIA (Comparative Example 10) and by the LTIA without the addition of the agents for suppressing non-specific reaction (Comparative Example 11) were approximately equivalent. Moreover, the measured values by the LTIA with the addition of the anti-C3d polyclonal antibody of the invention of the present application (Examples 4-1 to 4-5) were also approximately equivalent to those of Comparative Examples 10 and 11. Accordingly, it was found that the addition of the anti-C3d polyclonal antibody did not affect the measured values of the specimens exhibiting no non-specific reaction.
(2) The measurement results of the divergent specimens (specimen Nos. 16 and 17) were examined.

While the measured value of specimen No. 16 by the CLEIA (Comparative Example 10) was 346 U/mL, the measured value thereof by the LTIA without the addition of the agents for suppressing non-specific reaction (Comparative Example 11) was 1419 U/mL, which were largely different measured values. Furthermore, the measured value by the LTIA with the addition of HBR-1 (Comparative Example 12) was 1216 U/mL, which was largely different from that of Comparative Example 10. On the other hand, the measured value by the LTIA with the addition of 25 µg/mL of the anti-C3d polyclonal antibody of the invention of the present application (Example 4-1) was 352 U/mL, showing a tendency towards a value closer to that of Comparative Example 10. Similarly, the measured values by the LTIA with the addition of 50 and 500 µg/mL of the anti-C3d polyclonal antibody of the invention of the present application (Examples 4-2 and 4-5) were 395 and 374 U/mL, showing a tendency towards values closer to that of Comparative Example 10. Furthermore, similar results were obtained also for specimen 17. The anti-C3d polyclonal antibody of the invention of the present application exhibited an effect of suppressing non-specific reaction at least in the range of 25 to 500 µg/mL.

### [Reference Example 1] (Production of Complement C3d-Specific Monoclonal Antibodies)

### 1. Materials

(1) Complete Freund's adjuvant: manufactured by FUJIFILM Wako Pure Chemical Corporation, 014-09541
(2) Incomplete Freund's adjuvant: manufactured by FUJIFILM Wako Pure Chemical Corporation, 011-09551
(3) Myeloma cells (SP2/O)
(4) RPMI1640, GlutaMAX: manufactured by GIBCO, 61870-036
(5) Fetal Bovine Serum (FBS): manufactured by BIOLOGICAL INDUSTRIES, 04-001-1A
(6) HAT medium: manufactured by Cosmo Bio Co., Ltd., 16213004
(7) 96-Well plate: NUNC,167008
(8) HRP-labeled goat anti-mouse IgG (H & L): manufactured by Southern Biotech, 1031-05
(9) Complement C3d, human: manufactured by Sigma-Aldrich, 204870
(10) Complement C3d Goat anti-Human Polyclonal Antibody: manufactured by LifeSpan BioSciences, LS-C147220
(11) Biotin Labeling Kit-NH2: manufactured by Dojindo Molecular Technologies, LK03
(12)C3d, Human, clone 3: manufactured by Hycult Biotech, HM2198

### 2. Production of Hybridomas Producing Monoclonal Antibody against Human-Derived Complement C3d

### (2-1) Immunization of Animals

As an immunogen, a commercial human-derived complement C3d (manufactured by Sigma-Aldrich) was used. An emulsion was prepared by mixing equivalent amounts of the immunogen which was diluted with a 20 mM phosphate buffer containing 150 mM sodium chloride (pH7.2; called PBS below) and complete Freund's adjuvant, and 20 µg per mouse of the emulsion was injected to Balb/cAJcl mice. Furthermore, using incomplete Freund's adjuvant from the second injection, injection of 10 µg per mouse was repeated four times (five times in total including the first immunization) at intervals of two weeks. The antibody potencies of the antiserum obtained by taking blood from the tail vein were measured by the antigen-immobilized ELISA described below.

### (2-2) Evaluation of Antibody Potencies of Serum of Immunized Animals (Antigen-Immobilized ELISA)

The presence of an antibody against complement C3d in the serum of the immunized animals was observed by an immunogen-immobilized ELISA (antigen-immobilized ELISA). The details of the antigen-immobilized ELISA are as follows.

### (2-2-1) Production of Antigen-Immobilized ELISA Plate

The immunogen, complement C3d, was dissolved in PBS at 0.5 µg/mL, and 50 µL of the solution was dispensed to the wells of a 96-well microplate. The plate was left still at room temperature for two hours.

After washing the wells three times with 400 µL of PBS containing 0.05% Tween (registered trademark) 20 (called PBST below), 100 µL of PBST containing 1% bovine serum albumin (called BSA-PBST below) was added, and the wells were blocked at room temperature for an hour. The plate was used as an ELISA plate.

### (2-2-2) Antigen-Immobilized ELISA

After removing BSA-PBST from the wells of the ELISA plate, 50 µL of the antiserum of the immunized animals which was diluted for serial dilution with BSA-PBST and serum of an unimmunized animal were added to the wells, and the plate was left still at room temperature for an hour. After washing the wells three times with 400 µL of PBST, 50 µL of HRP-labeled anti-mouse IgG (H&L) which was diluted 9500-fold with BSA-PBST was dispensed to the wells, and the plate was left still at room temperature for an hour. After washing the wells three times with 400 µL of PBST, 50 µL of a citrate buffer (pH5.0) containing 0.2% o-phenylenediamine and 0.02% hydrogen peroxide was added, and the plate was left at room temperature for 10 minutes. Then, the enzymatic reaction was stopped by adding 50 µL of 1.5N sulfuric acid, and the absorbances at a wavelength of 492 nm were measured. From the mice in which an increase in the antibody potency was observed as a result of the measurement, spleens and lymph nodes were taken out, and spleen- and lymph node-derived cells were prepared and used for cell fusion.

### (2-3) Cell Fusion

Either the spleen-derived cells or the lymph node-derived cells and myeloma cells were mixed at a cell ratio of one to one, and the cells were fused by the electric pulse method. The fused cells were suspended in HAT medium and cultured in a CO₂ incubator at 37°C at 5% CO₂ for eight days, and fused cells (hybridomas) were thus obtained.

### (2-4) Selection of Hybridomas (Sandwich ELISA)

Hybridomas were selected by a sandwich ELISA using an anti-complement C3d polyclonal antibody. The details of the sandwich ELISA are as follows.

### (2-4-1) Production of Sandwich ELISA Plate

An anti-mouse IgG antibody was dissolved in PBS at 5 µg/mL, and 50 µL of the solution was dispensed to the wells of a 96-well microplate. The plate was left still at room temperature for two hours.

After washing the wells three times with 400 µL of PBS containing 0.05% Tween (registered trademark) 20 (called PBST below), 100 µL of PBST containing 1% bovine serum albumin (called BSA-PBST below) was added, and the wells were blocked at room temperature for an hour. The plate was used as an ELISA plate.

### (2-4-2) Sandwich ELISA

After removing BSA-PBST from the wells of the ELISA plate, the culture supernatants of the hybridomas were added to the wells, and the plate was left still at room temperature for an hour. After washing the wells three times with 400 µL of PBST, 50 µL of complement C3d which was diluted to 50 ng/mL with BSA-PBST was dispensed to the wells, and the plate was left still at room temperature for an hour. After washing the wells three times with 400 µL of PBST, 50 µL of an anti-complemen C3d polyclonal antibody (manufactured by LifeSpan BioSciences) which was labeled with biotin using a biotin labeling kit (manufactured by Dojindo Molecular Technologies) and which was diluted to 0.8 µg/mL with BSA-PBST was dispensed to the wells, and the plate was left still at room temperature for an hour. After washing the wells three times with 400 µL of PBST, 50 µL of HRP-labeled streptavidin which was diluted to 0.2 µg/mL with BSA-PBST was dispensed to the wells, and the plate was left still at room temperature for 30 minutes. After washing the wells three times with 400 µL of PBST, 50 µL of a citrate buffer (pH5.0) containing 0.2% o-phenylenediamine and 0.02% hydrogen peroxide was added, and the plate was left at room temperature for 10 minutes. Then, the enzymatic reaction was stopped by adding 50 µL of 1.5N sulfuric acid, and the absorbances at a wavelength of 492 nm were measured. The wells which had high absorbances as a result of the measurement were selected as wells with anti-complement C3d antibody-producing hybridomas (positive wells). Here, a commercial mouse-derived anti-C3d monoclonal antibody (manufactured by Hycult Biotech) was used at a concentration of 1 µg/mL as a positive control, instead of the culture supernatants of the hybridomas.

### (2-5) Cloning

Using the anti-complement C3d antibody-producing hybridoma strains selected in (2-4), single clones of the hybridomas were obtained, and the monoclonal antibodies were purified. The cloning was conducted by a conventional method (limiting dilution analysis). Positive wells were selected by a similar method to the sandwich ELISA described above, and five kinds of monoclonal antibody-producing hybridoma were obtained in the end. The monoclonal antibodies produced by the hybridomas S3520X (X=1 to 5) are called S3520X antibodies.

### [Example V: Suppression of Non-specific Reaction in LTIA: Measurement of sIL-2R]

A test for measuring the sIL-2R concentrations of samples using reagents by an LTIA with the addition of the anti-C3d monoclonal antibodies of the invention of the present application produced in Reference Example 1 is shown below. The same samples as those of Example IV were used.

### [Example 5] (Measurement by LTIA: Addition of Anti-C3d Monoclonal Antibodies)

Measurement was made by the same method as that in Comparative Example 5 except for adding the five kinds of anti-C3d monoclonal antibody produced in Reference Example 1 to the first reagent shown in Comparative Example 5 each at 100 µg/mL. The numbers of added clones are as follows. Example 5-1: clone No. S35201, Example 5-2: clone No. S35202, Example 5-3: clone No. S35203, Example 5-4 clone No. S35204, and Example 5-5: clone No. S35205. The measurement results are shown in Table 5.

**[Table 5]**

| | Measured sIL-2R Values (U/mL) of Samples | | | | | | | | |
|---|---|---|---|---|---|---|---|---|---|
| | Specimen No. | Measured IL-2R Value (U/mL) | | | | | | | |
| | | Cmp. Ex. 10 CLEIA | Cmp. Ex. 11 LTIA | Cmp. Ex. 12 LTIA | Example 5-1 LTIA | Example 5-2 LTIA | Example 5-3 LTIA | Example 5-4 LTIA | Example 5-5 LTIA |
| | | - | Without Additives | Addition of 100 *µ* g/mL of HBR-1 | Addition of 100 *µ* g/mL of Anti-C3d Monoclonal Antibody | | | | |
| | | | | | Clone No. S35201 | Clone No. S35202 | Clone No. 535203 | Clone No. 535204 | Clone No. S35205 |
| Control Specimen | 12 | 1400 | 1378 | 1421 | 1375 | 1359 | 1368 | 1383 | I 1382 |
| | 24 | 450 | 513 | 528 | 487 | 487 | 460 | 489 | 466 |
| | 25 | 718 | 756 | 782 | 722 | 753 | 726 | 761 | 751 |
| Divergent Specimen | 16 | 346 | 1419 | 1216 | 377 | 366 | 339 | 368 | 350 |
| | 17 | 171 | 474 | 349 | 268 | 307 | 277 | 287 | 262 |

### (Results and Discussion)

From the results of Comparative Examples 10 to 12 and Examples 5-1 to 5-5, the effects of the invention of the present application were discussed.
(1) The measurement results of the control specimens (specimen Nos. 12, 24 and 25) were examined.
   The measurement results of Comparative Examples 10 and 11 are as described in the discussion in Example IV Moreover, the measured values by the LTIA with the addition of the anti-C3d monoclonal antibodies of the invention of the present application (Examples 5-1 to 5-5) were also approximately equivalent to those of Comparative Examples 10 and 11. Accordingly, it was found that the addition of the anti-C3d monoclonal antibodies did not affect the measured values of the specimens exhibiting no non-specific reaction.
(2) The measurement results of the divergent specimens (specimen Nos. 16 and 17) were examined.

The measurement results of Comparative Examples 10, 11 and 12 are as described in the discussion in Example IV On the other hand, the measured value by the LTIA with the addition of 100 µg/mL of anti-C3d monoclonal antibody clone No. S35201 of the invention of the present application (Example 5-1) was 377 U/mL, showing a tendency towards a value closer to that of Comparative Example 10. Similarly, the measured values by the LTIA with the addition of 100 µg/mL of the anti-C3d monoclonal antibodies of the invention of the present application, clone Nos. S35202 to S35205 (Examples 5-2 to 5-5) were 339 to 368 U/mL, showing a tendency towards values closer to that of Comparative Example 10. Furthermore, similar results were obtained also for specimen 17. The anti-C3d monoclonal antibodies of the invention of the present application exhibited an effect of suppressing non-specific reaction.

From the above results, the effect of the anti-C3d antibodies of the invention of the present application of suppressing sample-derived non-specific reaction could be obtained both with polyclonal antibodies and monoclonal antibodies. It is extremely important to make accurate measurement in an immunoassay, and that suppression of non-specific reaction which still could not be suppressed even with a commercial agent for suppressing non-specific reaction has become possible by the invention has great significance.

### INDUSTRIAL APPLICABILITY

According to the invention, accurate immunoassay has become possible by conducting the immunoreaction in the presence of an anti-C3 antibody even when a non-specific factor is contained in a sample.

## Claims

1. A immunoassay method for immunologically measuring an analyte in a sample comprising conducting immunoreaction in the presence of an anti-C3 antibody.

2. The immunoassay method according to claim 1, wherein the method is a homogeneous method or a heterogeneous method.

3. The immunoassay method according to claim 1 or 2, wherein the homogeneous method is a latex immunoagglutination measurement method.

4. The immunoassay method according to claim 3, comprising
bringing an analyte in a sample and the anti-C3 antibody into contact with each other in a solution,
adding latex particles carrying a specific binding partner for the analyte to the solution and
optically detecting the degree of agglutination of the latex particles in the solution.

5. The immunoassay method according to any one of claims 1 to 4, wherein the anti-C3 antibody is any one or more selected from the group consisting of an anti-iC3b antibody, an anti-C3dg antibody, an anti-C3d antibody and an anti-C3b antibody.

6. A method for suppressing non-specific reaction in an immunoassay method for measuring an analyte in a sample immunologically, comprising conducting immunoreaction in the presence of an anti-C3 antibody.

7. The method for suppressing non-specific reaction according to claim 6, wherein the immunoassay method is a homogeneous method or a heterogeneous method.

8. The method for suppressing non-specific reaction according to claim 6 or 7, wherein the homogeneous method is a latex immunoagglutination measurement method.

9. The method for suppressing non-specific reaction according to claim 8, comprising
bringing an analyte in a sample and an anti-C3 antibody into contact with each other in a solution,
adding latex particles carrying a specific binding partner for the analyte to the solution and
optically detecting the degree of agglutination of the latex particles in the solution.

10. The immunoassay method according to any of claims 6 to 9, wherein the anti-C3 antibody is any one or more selected from the group consisting of an anti-iC3b antibody, an anti-C3dg antibody, an anti-C3d antibody and an anti-C3b antibody.

11. An immunoassay reagent comprising an anti-C3 antibody.

12. The immunoassay reagent according to claim 11, wherein a method of the immunoassay is a homogeneous method or a heterogeneous method.

13. The immunoassay reagent according to claim 12, wherein the homogeneous method is a latex immunoagglutination measurement method.

14. The immunoassay reagent according to any one of claims 11 to 13, wherein the anti-C3 antibody is any one or more selected from the group consisting of an anti-iC3b antibody, an anti-C3dg antibody, an anti-C3d antibody and an anti-C3b antibody.

15. An immunoassay reagent kit comprising an anti-C3 antibody.

16. The immunoassay reagent kit according to claim 15, wherein a method of the immunoassay is a homogeneous method or a heterogeneous method.

17. The immunoassay reagent kit according to claim 16, wherein the homogeneous method is a latex immunoagglutination measurement method, and the immunoassay reagent kit comprises:
(1) a first reagent containing the anti-C3 antibody; and
(2) a second reagent containing latex particles carrying a specific binding partner for an analyte.

18. The immunoassay reagent according to any of claims 15 to 17, wherein the anti-C3 antibody is any one or more selected from the group consisting of an anti-iC3b antibody, an anti-C3dg antibody, an anti-C3d antibody and an anti-C3b antibody.

19. An agent for suppressing non-specific reaction in an immunoassay method comprising an anti-C3 antibody as an active ingredient.

20. The agent for suppressing non-specific reaction in an immunoassay method according to claim 19, wherein the anti-C3 antibody is any one or more selected from the group consisting of an anti-iC3b antibody, an anti-C3dg antibody, an anti-C3d antibody and an anti-C3b antibody.
